# NEW EUROPEAN PATENT SPECIFICATION

(11) **EP 1 222 469 B2**
(45) Date of publication and mention of the opposition decision: **19.08.2009**
(45) Mention of the grant of the patent: 04.01.2006
(21) Application number: 00969458.9
(22) Date of filing: 03.10.2000
(51) Int. Cl.: G01N 33/94, C12Q 1/18

(54) **ONE STEP TEST TO DETECT ANTIMICROBIAL RESIDUES IN EGGS**
EIN-SCHRITTTEST ZUM NACHWEIS VON ANTIMIKROBIELLEN RÜCKSTÄNDEN IN EIERN
DETECTION DE RESIDUS ANTIMICROBIENS DANS LES OEUFS

(30) Priority: 04.10.1999 EP 99203264
(43) Date of publication of application: 17.07.2002
(73) Proprietor: DSM IP Assets B.V., 6411 TE Heerlen (NL)
(72) Inventor: LANGEVELD, Pieter, Cornelis, NL-2622 BP Delft (NL); STARK, Jacobus, NL-3069 ZM Rotterdam (NL)
(74) Representative: Matulewicz, Emil Rudolf Antonius
(86) International application number: PCT/EP2000/009872
(87) International publication number: WO 2001/025795

(56) References cited:
- US-A- 5 354 663
- US-A- 5 489 532
- US-A- 5 494 805
- CHEMICAL ABSTRACTS, vol. 89, no. 23, 4 December 1978 (1978-12-04) Columbus, Ohio, US; abstract no. 195540, XP002132528 cited in the application & J.M. INGLIS ET AL. : " Determination of streptomycin residues in eggs and stability of residues after cooking" JOURNAL OF THE ASSOCIATION OF OFFICIAL ANALYTICAL CHEMISTS, vol. 61, no. 5, 1978, pages 1098-1102, New Brunswick NJ USA
- 'Operator's manual', 01 January 1997, CHARM SCIENCES INC. article 'Charm Inhibition Assay for antimicrobial Drugs in Eggs'
- WALSTRA P., JENNESS, R. DAIRY CHEMISTRY AND PHYSICS 01 January 1984, pages 166 - 167
- NELSON, D.L., COX M.M.: 'Part III Structure and Catalysis' LEHNINGER PRINCIPLES OF BIOCHEMISTRY 01 January 2000,
- DSM TECHNICAL BULLETIN 17 March 2006, pages 7 - 7
- DMS TECHNICAL BULLETIN 23 June 2006, pages 8 - 8
- BIOLOGICAL ABSTRACTS, 01 January 2003, Philadelphia, PA, US; HALL, H.C. ET AL: 'Antibiotic residue surveillance at the Veterinary Services Labaratory' page 1-15;
- 'Label instructions for negative control standard 1999', 01 August 2002, CHARM CATALOGUE page 1-3

## Description

### Field of the invention

The present invention relates to a novel method for the rapid detection of the presence or absence of antimicrobial residues in eggs.

### Backctround of the invention

The presence of antimicrobial residues in food and feed is a growing concern among the consumers due to health-related problems and the increase of drug resistant bacteria. Antibiotics are not only applied as medication, but certainly in case of poultry also widely used as antimicrobial growth promoting substances. It is well known that concentrations of antimicrobial residues in eggs may be high. In most countries, such as the countries of the European Union, Canada and the United States, Maximum Residue Levels (MRL) are regulated by legislation.

Test methods to detect antimicrobial residues in body liquids such as milk or urine such as microbial inhibition tests (e.g. agar diffusion tests) or methods making use of selective binders (e.g. antibodies or tracers) are well known. Examples of microbiological test methods have been described in GB-A-1467439, EP 0005891, DE 3613794, CA 2056581, EP 0285792 and US 5494805. These documents all deal with ready to use tests that make use of a test organism. The test organism is mostly imbedded in an agar medium, which may contain an indicator, a buffer solution, nutrients and substances to change the sensitivity for certain antimicrobial compounds in a positive or negative way.

Examples of suitable test organisms are strains of *Bacillus, Streptococcus* or *E.coli.* ln general, the principle of these tests is that when antibacterial compounds are present in a sample at a concentration sufficient to inhibit the growth of the test organism the color of an acid/base or redox indicator will stay the same. However when no inhibition occurs, growth of the test organism is accompanied by the formation of acid or reduced metabolites leading to a change in the colour of the indicator.

These test methods are suitable for the detection of antimicrobial residues in body liquids. However up to now detection of antimicrobial residues in eggs was not possible due to the presence of antimicrobial substances, such as lysozyme, which are naturally present in high concentrations in eggs. These inhibiting substances show inhibitory activity against the test micro-organism leading to false positive results.

In case of e.g. a milk or urine sample inhibiting substances such as lysozyme or lactoferrin can be inactivated by heating the sample, e.g. at 80°C for 10 minutes (Vermunt et. al., Netherlands Milk and Dairy Journal 47: (1) 31 - 40 (1993)), or by using well known dialysis methods (van Wall, Archiv für Lebensmittelhygiene 29: (6) 235 (1978)). After this pre-treatment the liquid sample can be used for further testing simply by following the procedures of the test. In case of a Delvotest® type of test (EP 0005891) the liquid sample can be added directly to the test, after which the test is incubated.

However heating of an egg sample at temperatures sufficient to inactivate inhibiting substances of the egg, such as lysozyme, always leads to coagulation of the sample. It was believed that such samples are not suitable for further processing anymore.

Up to now after heating at a temperature sufficient to inactivate antimicrobial substances other than antimicrobial residues, the antimicrobial residues to be detected have to be extracted from the coagulated egg sample. These extraction methods not only cost a lot of time and extra handling but even worse always lead to loss of at least part of the antimicrobial residues, if present in the sample (Inglis et. al., Journal for the Association of Official Analytical Chemists 61: (5) 1098 - 1102 (1978); Katz et. al., Journal of the Association of Official Analytical Chemists 61: (5) 1103 - 1106 (1978); Janetschke et. al., Monatshefe für Veterinaermedizin 34: (21) 824 - 826 (1979); Steiner, Monatshefe für Veterinarmedizin 45: (11) 382 - 386 (1990)). This may lead to false negative results and therefore antibiotics in consumer eggs, which of course is unacceptable from a health point of view. Moreover laboratories executing studies concerning the presence or absence of antimicrobial residues in foods are limited by the time available to execute these studies. With the present time consuming methods only a very limited amount of egg samples can be examined. Further, these assays can only be executed in well-equipped laboratories and by well-educated persons, which is also a limiting factor.

It can be concluded that up to now no suitable test method for the detection of antimicrobial residues in egg samples is available. The present methods are unreliable, time consuming and may lead to both false positive and false negative results, which in turn leads to unacceptable amounts of antibiotics in the food chain and to economic losses.

### Detailed description of the invention

The present invention provides a reliable and simple to carry out, one-step test for the detection of antimicrobial residues in eggs.

Unexpectedly it has been found that when an egg sample is added to a test suitable for detecting antimicrobial residues and then is incubated for a sufficient time at a sufficient temperature to inactivate natural inhibiting compounds of the egg, the test can be incubated directly after heating to determine the presence or absence of antimicrobial residues.

It has been surprisingly found that antimicrobial residues diffuse directly from the coagulated egg sample into the test system. Thus additional extraction methods to obtain the antimicrobial residues from the coagulated egg sample are not required.

A process for determining the presence or absence of an anti-microbial residue in a sample of an egg, which process consists of:
(i) contacting the sample with a microbial inhibition test suitable for determining the presence or absence of an anti-microbial residue in the sample, wherein the test comprises a test organism, nutrients and one or more indicators present in an agar medium;
(ii) heating the contacted sample and test to a temperature of from 75°C to 85°C for from 10 to 15 minutes to inactivate a natural inhibiting compound present in the sample;
(iii) incubating the contacted sample and test; and
(iv) whereby the degree of growth or inhibition of growth of a test organism is determined indicating the absence or presence of the anti-microbial residue.
By natural inhibiting compounds are meant compounds which may disturb the test and which are naturally present in the sample such as naturally inhibiting compounds, for example lysozyme. Thus by disturbing or inhibiting is meant the behaviour of the compound on parts of the test, for example the test micro organism.

The exact time/temperature requirements depend on e.g. the condition of the sample (e.g. the starting temperature, the volume of the sample, whole egg, egg white or egg yolk); the type of test; or the microorganism used in the test (e.g. thermophilic *Bacillus* or *Streptomyces* species). Of course it should be taken care of that the heat treatment will not inactivate the antimicrobial residues to be detected. The heat treatment can be executed using any method known in the art, e.g. by heating in a water bath or by using an incubator as described below.

For example the test can be performed in the following way:
1. A sample of the egg is obtained by making a hole in the egg of e.g. approximately 1-2 square cm, prick the egg-yolk, place the egg with the hole down on a bottle, after the egg is empty the bottle is closed and the sample is homogenized by shaking. Alternatively of course any other method known in the art to obtain a sample of the total egg, egg white or egg yolk can be used;
2. Add a sufficient amount of the egg sample to be tested to a test using well known methods;
3. Heat the test, e.g. for approximately 10 minutes at 80° C, to inactivate the natural inhibiting substances (e.g. lysozyme), to coagulate the egg sample;
4. Incubate the test following the standard procedures of the test and read the result.

Any microbial inhibition test suitable for determining the presence or absence of antimicrobial residues may be used in a process of test kits of the invention. Examples are described in GB-A-1467439, EP-0005891, DE-3613794, CA-2056581, EP-028579 and US 5,494,805. Suitable tests are those in which selected sensitive microorganisms are used.

Examples of suitable microbial agar diffusion tests are tests in which species of *Bacillus, Streptococcus or E. coli* are used. Preferably thermophilic species, e.g. *Bacillus stearothermophilus* and *Streptococcus thermophilus* are used. Examples of preferred strains are *Bacillus stearothermophilus var. calidolactis* C953 (deposited with the Laboratory of Microbiology of the Technical University of Delft under the accession number LMD 74.1 in 1974 and with the Centraal Bureau voor Schimmelcultures (CBS), Baarn under the accession number CBS 760.83 in 1983 were the strain is available to the public) and *Streptococcus thermophilus T101* (DSM 4022, deposited on March 3, 1987). Both strains are very sensitive to antimicrobial compounds, especially chemotherapeutics such as sulfa compounds and antibiotics such as penicillins and tetracyclines. *E.coli* strains or other suitable gram-negative bacteria can be used for the detection of e.g. quinoiones.

*Bacillus stearothermophilus var. calidolactis C953* and *Streptococcus thermophilus T101* are fast growing and have the advantage that they are thermophilic. For example the optimum growth temperature of said *Bacillus* strain is from 50° to 70°C. The test organism is therefore very suitable for a test according to the invention as it is not killed by heating to inactivate the natural inhibiting compounds which may be present in the egg sample.

When the test organism is a *Bacillus* strain, it is preferably incorporated into the agar medium in the form of a spore suspension which may be prepared and incorporated into the agar medium prior to solidification by known methods (see for example GB-A-1467439). When the test organism is a *Streptococcus* strain, the bacteria are preferably incorporated into the agar medium in the form of bacterial cells which may be prepared according to known methods (see for example EP 0285792). The concentration of the test organism in the agar medium is preferably between 10⁵ and 10¹⁰ colony forming units per ml of agar medium.

Suitable nutrients to enable multiplication of the test organism in the absence of antimicrobial residues are for example assimilable carbon sources (e.g. lactose, glucose or dextrose), assimilable nitrogen sources (e.g. peptone) and sources of growth factors, vitamins and minerals (e.g. yeast extract).

The growth of the test microorganism can be detected using well known methods, preferably by colour change of the agar medium of the test sample. Typically a colour indicator, preferably an acid-base or a redox indicator, is used. Examples of suitable acid-base indicators include bromocresol purple and phenol red. Examples of suitable redox indicators include brilliant black, methylene blue, toludine blue and nile blue. Also combinations of two or more indicators can be used.

Optionally the sensitivity of the test may be altered by adding certain substances, by changing the test conditions such as pH or concentration of buffering substances or agar or by varying the ratio of the volumes of agar and egg sample. Examples of substances that may be added to the test system to change sensitivity are nucleosides such as adenosine, or antifolates such as trimethoprim, ormethoprim or tetroxoprim, which improve the sensitivity of the test organism to sulfa compounds. Salts of oxalic acid or hydrofluoric acid may be added to improve the sensitivity to tetracyclines. Cysteine may be added to diminish the sensitivity to penicillins.

The amount of egg sample (whole egg, egg white or egg yolk of any species, preferably poultry) to be added to the test depends on the test system. For microbial diffusion tests typically from 0.01 to 1.0 ml, preferably from 0.05 to 0.5 ml is added to the test using well-known methods. After addition of the egg sample the test is heated to inactivate the natural antimicrobial compounds present in the sample, e.g. lysozyme, of the egg sample. The test is heated for from 10 to 15 minutes at from 75°C to 85°C.

After the heat treatment the test is incubated following the instructions of the test manufacturor. The incubation time of the test is dependent on the circumstances. In case of an agar diffusion tests using *Bacillus stearothermophilus* the test is incubated in a water bath or block heater at from 60°C to 70°C, preferably at from 62°C to 65°C. Typically, results may be obtained after 1.5 to 4 hours, preferably from 2.5 to 3.5 hours.

Conventional microbial inhibition tests suitable for use in the present invention include the commercial products, Delvotest®, Premi®test and BR-test® (obtainable from DSM N.V. Holland) the ADM Copan®test (Copan, Italy) and the Charm®AIM test (Charm, USA). Inactivation of the natural inhibiting compounds present in the egg sample, e.g. lysozyme, is achieved by heating for from 10 to 15 minutes from 75° C to 85°C.

incubators suitable to execute the heat treatments as described in this invention can be constructed in such a way that after placing the test units in the incubator, heat and incubation treatments as described above can be done. The first heat treatment to inactivate the inhibiting compounds and optionally to form solid matrix and / or to activate the spores is executed at a higher temperature, after which the incubation of the test may continue at a lower temperature. Optionally after the incubation of the test the incubator can cool down to a temperature sufficient to stop the test.

An example of such an incubator is a block heater in which test units (e.g. ampoules) can be placed. For example in case of a conventional microbial agar diffusion test using a *Bacillus stearothermophilus* strain the incubator / block heater may contain a number of holes suitable for placing the test ampoules or test plates (e.g. Delvotest® or Premi®Test) therein. After placing the ampoules or plates the incubator heats the test to a temperature of from 75°C to 85°C for from 10 to 15 minutes after which the incubator turns to a lower temperature of from 62°C to 65°C for from 1.5 to 4 hours (incubation of the test). Of course the exact time temperature intervals depend on many factors and will differ per type of test. This invention includes all incubators capable to execute a pre-incubation at a certain temperature for a certain period of time directly followed by an incubation at a lower temperature for a certain period of time. Optionally after the incubation of the test the incubator can cool down to a temperature sufficient to stop the test.

The process described in this invention is very simple to carry out, so that persons who perform the test do not have to be specially educated or trained.

### Example 1

### Preparation of a whole egg sample

To obtain an egg sample for examination on the presence or absence of antimicrobial residues a hole of approximately 1-2 cm² was made in the egg, the egg yolk was pricked and the egg was placed with the hole down on a bottle allowing the egg white and egg yolk to drip into the bottle. After the egg was emptied, the bottle was closed and the sample was homogenized by shaking.

### Example 2

### Inactivation of natural inhibiting compounds of the egg and examining the samples on Delvotest®

Samples of 5 eggs (duplicate), which did not contain antimicrobial residues, were obtained according to the method described in Example 1. To inactivate the natural inhibiting compounds present in the egg sample, 100 µl of each of the 5 samples was added on Delvotest® ampoules. The test was produced according to the methods described in EP 0005891 with the nutrients present in the agar. After heating for 10 minutes at 80°C in a waterbath, the ampoules were immediately placed in a waterbath at 64°C and incubated following the instructions of the producer. After 140 minutes the colour of all tests turned from purple to yellow, indicating that no antimicrobial rediues were present.

Control samples were not heated at 80°C for 10 minutes, but directly placed on the ampoule. These tests remained purple for at least 4 hours.

These results clearly demonstrate that natural inhibiting compounds in the egg sample inhibited the test leading to false-positive results. When the sample was heated as described above, the activity of the natural inhibiting compounds was eliminated and no false-positive results were observed anymore.

### Example 3

### Determination of the sensitivity of the Delvotest® according to the method described in this invention using spiked samples

Egg samples were obtained according to the method described in Example I. The samples were spiked by adding Penicillin G (0 and 4 ppb) or Sulphadiazine (0 and 100 ppb). The egg samples were added to Delvotest® ampoules (see Example 2) according to the method described in this invention: heated for 10 minutes at 80°C, and then immediately placed in a waterbath at 64°C and incubated following the instructions of the manufacturer. The results were read as soon as the colour turned to yellow (after 140 minutes). The samples containing no Penicillin G (o ppb) or Sulphadiasine (0 ppb) were negative, while the samples spiked with 4 ppb Penicillin G and 100 ppb sulphadiazine remained purple (positive).

These results clearly demonstrate that the method descibed in this invention is suitable for detecting antimicrobial residues in egg samples.

## Claims

1. A process for determining the presence or absence of an anti-microbial residue in a sample of an egg, which process consists of:
(i) contacting the sample with a microbial inhibition test suitable for determining the presence or absence of an anti-microbial residue in the sample, wherein the test comprises a test organism, nutrients and one or more indicators present in an agar medium;
(ii) heating the contacted sample and test to a temperature of from 75°C to 85°C for from 10 to 15 minutes to inactivate a natural inhibiting compound present in the sample;
(iii) incubating the contacted sample and test; and
(iv) whereby the degree of growth or inhibition of growth of a test organism is determined indicating the absence or presence of the anti-microbial residue.

## Patentansprüche

1. Verfahren zum Bestimmen der Anwesenheit oder Abwesenheit eines antimikrobiellen Restes in einer Ei-Probe, wobei das Verfahren aus folgendem besteht:
(i) dem Kontaktieren der Probe mit einem mikrobiellen Inhibitionstest, der zum Bestimmen der Anwesenheit oder Abwesenheit eines antimikrobiellen Restes in der Probe geeignet ist, wobei der Test einen Testorganismus, Nährstoffe und einen oder mehrere Indikatoren, die in einem Agarmedium vorhanden sind, umfaßt,
(ii) dem Erwärmen der kontaktierten Probe und des Tests auf eine Temperatur von 75°C bis 85°C für 10 bis 15 Minuten, um eine natürliche inhibierende Verbindung, die in der Probe vorhanden ist, zu inaktivieren,
(iii)dem Inkubieren der kontaktierten Probe und des Tests, und
(iv) wodurch das Ausmaß des Wachstums oder der Inhibierung des Wachstums eines Testorganismus bestimmt wird, was die Abwesenheit oder Anwesenheit des antimikrobiellen Restes anzeigt.

## Revendications

1. Procédé pour déterminer la présence ou l'absence d'un résidu antimicrobien dans un échantillon d'un oeuf, lequel procédé consiste en :
(i) la mise en contact de l'échantillon avec un test d'inhibition microbien adéquat pour la détermination de la présence ou de l'absence d'un résidu antimicrobien dans l'échantillon, le test comprenant un organisme test, des nutriments et un ou plusieurs indicateurs présents dans un milieu gélosé ;
(ii) le chauffage de l'échantillon et du test en contact à une température allant de 75°C à 85°C pendant 10 à 15 minutes pour inactiver un composé inhibiteur naturel présent dans l'échantillon ;
(iii) l'incubation de l'échantillon et du test en contact, et
(iv) le degré de croissance ou l'inhibition de la croissance d'un organisme test étant déterminé(e) en indiquant l'absence ou la présence d'un résidu antimicrobien.
